# EUROPÄISCHE PATENTSCHRIFT

(11) **EP 1 778 112 B1**
(45) Veröffentlichungstag und Bekanntmachung des Hinweises auf die Patenterteilung: **29.08.2012**
(21) Anmeldenummer: 05745726.9
(22) Anmeldetag: 25.05.2005
(51) Int. Cl.: A61B 18/14

(54) **ELEKTROCHIRURGISCHES INSTRUMENT**
ELECTROSURGICAL INSTRUMENT
INSTRUMENT ELECTRO-CHIRURGICAL

(30) Priorität: 28.06.2004 DE 102004031141
(43) Veröffentlichungstag der Anmeldung: 02.05.2007
(73) Patentinhaber: ERBE Elektromedizin GmbH, 72072 Tübingen (DE)
(72) Erfinder: FISCHER, Klaus, 72202 Nagold (DE)
(74) Vertreter: Bohnenberger, Johannes
(86) Internationale Anmeldenummer: PCT/EP2005/005680
(87) Internationale Veröffentlichungsnummer: WO 2006/000280

(56) Entgegenhaltungen:
- US-A- 5 891 142
- US-B1- 6 273 887
- US-B1- 6 364 879

## Beschreibung

Die Erfindung betrifft ein elektrochirurgisches Instrument nach dem Oberbegriff des Patentanspruches 1. Ein solches Gerät ist zum Beispiel aus US-B1-6 273 887 bekannt.

Elektrochirurgische Instrumente werden seit vielen Jahren in der Hochfrequenz-Chirurgie eingesetzt, um biologisches Gewebe zu koagulieren oder zu schneiden. Bei einer Koagulation wird ein hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden.

Für einen Schneidprozess ist eine hohe Stromdichte erforderlich, so dass ein explosionsartiges Verdampfen der Gewebeflüssigkeit und ein Aufreißen der Zellmembranen das Gewebe vollständig durchtrennen.

Der Einsatz bipolarer Instrumente gewinnt immer mehr an Bedeutung, da geringere Stromstärken als bei monopolaren Instrumenten nötig sind. Von Vorteil ist es insbesondere, dass der Stromweg zwischen den Elektrodenteilen bipolarer Instrumente kalkulierbar ist und nicht weite Strecken durch den Körper des Patienten verläuft.

Bipolare Instrumente weisen im Wesentlichen zwei gelenkig miteinander verbundene Klemmenteile auf, wobei an deren proximalen Enden Griffeinrichtungen zur Handhabung der Klemmenteile vorgesehen sind. An distalen Enden der Klemmenteile befinden sich Elektrodenteile zum Fassen von Gewebe und zum Durchleiten eines Koagulationsstromes durch das Gewebe. Eine von einem HF-Generator erzeugte HF-Spannung und der dadurch zur Verfügung stehende HF-Strom wird über Stromzuführungseinrichtungen zu den Elektrodenteilen des bipolaren Instrumentes geleitet. Um einen Kurzschluss bei Berührung der beiden Elektrodenteile zu vermeiden, weisen die bekannten Instrumente eine an den Branchen angebrachte Vorrichtung zur Vermeidung eines Kurzschlusses auf, wobei die Elektrodenteile bei geschlossenem Instrument stets einen Abstand zueinander aufweisen.

Problematisch bei den bekannten Vorrichtungen zur Vermeidung eines Kurzschlusses ist es nun, dass diese den Abstand zwischen den Elektrodenteilen nur mittelbar definieren, weil sie entfernt von den Elektrodenteilen angebracht sind. So müssen z. B. die Längenverhältnisse der Branchen berücksichtigt werden, um den geeigneten Abstand festzulegen. Damit wird das Abstimmen von Abstand und benötigter HF-Spannung deutlich erschwert.

Der Erfindung liegt daher die Aufgabe zugrunde, ein elektrochirurgisches Instrument der eingangs genannten Art dahin gehend weiterzubilden, dass eine Abstimmung zwischen dem Abstand zwischen den Elektrodenteilen und der benötigten HF-Spannung mit erhöhter Zuverlässigkeit durchführbar ist.

Diese Aufgabe wird durch ein elektrochirurgisches Instrument nach Patentanspruch 1 gelöst.

Insbesondere wird die Aufgabe durch ein elektrochirurgisches Instrument gelöst, das zwei gelenkig miteinander verbundenen Branchen umfasst, die entsprechend einem Klemmwerkzeug betätigbar sind. Ferner umfasst das Instrument Elektrodenteile an distalen Enden der Branchen zum Fassen von Gewebe und zum Durchleiten eines Koagulationsstromes durch das Gewebe zu dessen Koagulation sowie Stromzuführungseinrichtungen zum Zuführen des Koagulationsstromes zu den Elektrodenteilen von einem HF-Generator. Es ist mindestens eine Vorrichtung zur Vermeidung eines Kurzschlusses derart angebracht und ausgebildet, dass die Elektrodenteile sich nicht berühren können, wobei die Vorrichtung zur Vermeidung eines Kurzschlusses an den Elektrodenteilen ausgebildet ist.

Ein wesentlicher Punkt der Erfindung liegt darin, dass die Vorrichtung zur Vermeidung eines Kurzschlusses nun unmittelbar an der Stelle angeordnet ist, an der der wirksame Abstand tatsächlich vorhanden sein muss, also zwischen den Elektrodenteilen. Gleichzeitig wird eine Koagulation nicht behindert, weil das Gewebe aufgrund Wärmeleitung auch an einer Kontaktstelle von Gewebe und Vorrichtung koagulieren kann. Insofern ist bei einem Koagulationsvorgang eine Funkenbildung zwischen den Elektrodenteilen zuverlässig vermeidbar.

Zur Vermeidung eines Kurzschlusses weist die Vorrichtung ein als isolierender Abschnitt vorgesehenes Distanzelement an mindesten einem Elektrodenteil auf. Damit wird bereits durch das Distanzelement der Kurzschluss zwischen den Elektrodenteilen vermieden, sollten diese sich berühren.

An den isolierenden Abschnitt angrenzend ist mindestens ein erster Schneidabschnitt an mindestens einem der Elektrodenteile derart angeordnet, insbesondere mit einem in Bezug auf die Koagulationselektroden verringerten Abstand zur Gegenelektrode ausgebildet, dass bei einer Erhöhung der Spannung des Koagulationsstromes von dem ersten Schneidabschnitt ausgehend ein Lichtbogen zum Durchtrennen des Gewebes entsteht. Der Schneidabschnitt ist als ein sich im Verhältnis zu dem Elektrodenteil hervorstehender Bereich an dem Elektrodenteil angeordnet und steht aus diesem hervor. Das Elektrodenteil weist dann neben dem Schneidabschnitt einen expliziten Koagulationsabschnitt auf. Das den Koagulationsabschnitt und den Schneidabschnitt ausbildende Elektrodenteil kann während eines Koagulationsvorganges über seinen gesamten Flächenbereich, d. h. sowohl über den Flächenbereich des Koagulationsabschnittes als auch über den Flächenbereich des Schneidabschnittes, als Koagulationselektrode wirken, während der hervorstehende Schneidabschnitt allein für einen späteren Schneidvorgang zur Verfügung steht.

Durch den Schneidabschnitt ist gewährleistet, dass der Lichtbogen nur an diesem entsteht, während ein Durchschlag an dem restlichen Elektrodenabschnitt zu der gegenüberliegenden Elektrode aufgrund eines zu großen Abstandes zwischen den Elektrodenteilen nicht auftreten kann. Somit ist ein und dasselbe Instrument sowohl zum Koagulieren als auch zum Schneiden verwendbar und ein Instrumentenwechsel kann zugunsten eines zügigen Operationsverlaufs vermieden werden.

Das Distanzelement kann dabei sowohl linienförmig als auch punktförmig ausgebildet sein. Das linienförmig ausgebildete Distanzelement zieht sich dann beispielsweise in Richtung des Branchenverlaufs, mittig an dem Elektrodenteil angeordnet, über das gesamte Elektrodenteil und bildet demgemäß eine Kante aus. Vorteilhafterweise wird auf diese Weise die gleichmäßige Ausbildung eines Lichtbogens ermöglicht und ein ebenmäßiger Schnitt gewährleistet. Ein punktförmig ausgebildetes Distanzelement ist einfach herstellbar, vermeidet zuverlässig die Entstehung des Kurzschlusses zwischen den Elektrodenteilen und gewährleistet aufgrund Wärmeleitung eine sichere Koagulation auch an der Kontaktstelle zwischen Gewebe und Distanzelement. Mehrere, auf dem entsprechenden Elektrodenteil, z. B. an den jeweiligen Enden des Elektrodenteils angeordnete, punktförmig ausgebildete Distanzelemente, verhindern zuverlässig den Kurzschluss und beeinträchtigen weder den Koagulations- noch den Schneidvorgang.

Vorzugsweise ist an dem Elektrodenteil, das dem Elektrodenteil mit dem isolierenden Abschnitt gegenüberliegt, ein zweiter Schneidabschnitt ausgebildet. Dies ist insbesondere dann von Vorteil, wenn der dem zweiten Schneidabschnitt gegenüberliegende isolierende Abschnitt unmittelbar an dem Elektrodenteil angeordnet ist, ohne dass das Elektrodenteil einen expliziten ersten Schneidabschnitt aufweist. In dieser Ausführungsform ist der isolierende Abschnitt vorzugsweise kleiner als der gegenüberliegende Schneidabschnitt ausgelegt, so dass ein Lichtbogen um Randbereiche des isolierenden Abschnitts zu dem gegenüberliegenden Schneidabschnitt ausgebildet werden kann. Damit ist ein präziser Schnitt erreichbar.

Alternativ ist es möglich, den Schneidabschnitt an beiden sich gegenüberliegenden Elektrodenteilen auszubilden. Damit sind der Bereich für die Lichtbogenentstehung und der Schnittverlauf äußerst präzise definiert.

Vorzugsweise weist die Vorrichtung zur Vermeidung eines Kurzschlusses mindestens einen isolierenden Abschnitt auf, der innerhalb eines Elektrodenteils ausgebildet ist. Ein als Schneidabschnitt vorgesehenes Distanzelement ist dann an dem dem isolierenden Abschnitt gegenüberliegenden Elektrodenteil ausgebildet. Der Schneidabschnitt ist derart angeordnet, dass er bei Zusammenführung der Branchen ausschließlich den isolierenden Abschnitt berührt. Vorteilhafterweise wird auch hier eine präzise Schnittführung vorgegeben, da der Lichtbogen zwischen dem eingearbeiteten Abschnitt und dem Schneidabschnitt entsteht. Der isolierende Abschnitt ist bei dieser Ausführungsform vor Stößen oder dergleichen mechanischen Belastungen und im Wesentlichen auch vor dem Lichtbogen geschützt.

Der isolierende Abschnitt kann in dieser Ausführungsform bündig mit einer Elektrodenfläche des den isolierenden Abschnitt aufweisenden Elektrodenteils ausgebildet sein. Damit lässt sich das Elektrodenteil nach einer Behandlung einfach und sicher reinigen.

Alternativ ist es möglich, den isolierenden Abschnitt vertieft in dem entsprechenden Elektrodenteil anzuordnen, so dass das Elektrodenteil eine Aussparung aufweist. Der an dem gegenüberliegenden Elektrodenteil ausgebildete Schneidabschnitt ist damit mindestens teilweise in die Aussparung 18c versenkbar, so dass sich während eines Schneidvorganges ein Lichtbogen innerhalb der Aussparung hin zu dem Schneidabschnitt ausbilden kann. Umliegendes Gewebe ist so vor Verbrennungen geschützt, gleichzeitig ist eine präzise Schnittlinie definierbar.

In einer bevorzugten Ausführungsform ist der isolierende Abschnitt symmetrisch zum ersten Schneidabschnitt und/oder zweiten Schneidabschnitt an den jeweiligen Elektrodenteilen angeordriet. Eine symmetrische Anordnung der zusammenwirkenden Abschnitte gewährleistet einen gleichmäßig verlaufenden Lichtbogen an Randbereichen des Distanzelements, so dass ein ebenmäßiger Schnittverlauf ermöglicht wird.

Eine mögliche Ausführungsform sieht vor, dass das Distanzelement derart ausgebildet ist, dass ein mechanisches Schneiden durchführbar ist. Vorzugsweise weist das Distanzelement dann eine Schneidkante auf, die sich für das mechanische Schneiden eignet. Mit entsprechender Kraftaufbringung durch den Chirurgen kann dann, nach einem Koagulationsvorgang und ohne einen Instrumentenwechsel vornehmen zu müssen, das Gewebe vollständig durchtrennt werden. Damit wird eine besonders sanfte Behandlung des Gewebes erzielt.

In einer nicht beanspruchten Ausführungsform ist der Schneidabschnitt als Kante mit einem im Wesentlichen dreieckförmigen Querschnitt an dem mindestens einen Elektrodenteil ausgebildet. Ein dreieckförmiger Querschnitt erlaubt den sukzessiven Übergang von einem großen Flächenbereich des Elektrodenteils bis zu deren kantenförmiger Verjüngung. Der sanfte Übergang ist in besonderem Maße geeignet, das gesamte Elektrodenteil bei ausreichender Gewebedicke als Koagulationselektrode einzusetzen, weil der gesamte Flächenbereich und das Gewebe miteinander in Kontakt gebracht werden können. Vorteilhafterweise ist der Schneidabschnitt dieser nicht beanspruchten Ausführungsform als Kante mit einem im Wesentlichen abgerundeten oder kreisförmigen Querschnitt an dem mindestens einen Elektrodenteil ausgebildet. An einem Übergang zwischen dem expliziten Koagulationsabschnitt und dem Schneidabschnitt ist der Schneidabschnitt bevorzugt abgeflacht ausgebildet, so dass eine sichere Befestigung des Schneidabschnittes an dem entsprechenden Elektrodenteil gewährleistet ist. Bei dieser Ausführungsform steht eine relative große Elektrodenfläche für den Koagulationsvorgang zur Verfügung, während der als Kante ausgebildete Schneidabschnitt bei genügend großer Gewebedicke kaum ins Gewicht fällt. In einem fortgeschrittenen Operationsstadium hingegen und bei ausreichender Nähe gegenüberliegender Elektrodenteile des elektrochirurgischen Instruments lässt sich aufgrund der kantenförmigen Ausgestaltung des Schneidabschnittes die Stromdichte derart erhöhen, dass ein Schneidvorgang ermöglicht wird.

Vorzugsweise ist der isolierende Abschnitt aus einem abbrandfesten Material ausgebildet. Damit ist eine zuverlässige Verschleißfestigkeit gegenüber dem Lichtbogen gegeben.

In einer bevorzugten Ausführungsform besteht der isolierende Abschnitt aus einer Keramik. Vorteilhafterweise weisen Keramiken eine hohe Korrosionsbeständigkeit und eine hohe Verschleißfestigkeit gegenüber dem Lichtbogen und auch gegenüber mechanischer Belastung auf.

Nachfolgend wird die Erfindung anhand von Ausführungsbeispielen beschrieben, die anhand der Abbildungen näher erläutert werden. Hierbei zeigen
- - Fig. 1a: eine schematisch dargestellte Elektrodenanordnung in einer ersten Ausführungsform während einer Koagulationsphase;
- - Fig. 1b: die Elektrodenanordnung gem. Fig. 1a nach Beendigung der Koagulationsphase;
- - Fig. 1c: die Elektrodenanordnung gem. Fig. 1a während einer Schneidphase;
- - Fig. 2: eine schematisch dargestellte Elektrodenanordnung in einer zweite Ausführungsform;
- - Fig. 3: eine schematisch dargestellte Elektrodenanordnung in einer dritten Ausführungsform;
- - Fig. 4: eine schematisch dargestellte Elektrodenanordnung in einer vierten Ausführungsform;
- - Fig. 5: eine schematisch dargestellte Elektrodenanordnung in einer fünften Ausführungsform;
- - Fig. 6: eine schematisch dargestellte Elektrodenanordnung in einer sechsten Ausführungsform;
- - Fig. 7: ein schematisch dargestelltes elektrochirurgisches Instrument mit einer erfindungsgemäßen Elektrodenanordnung.

In der nachfolgenden Beschreibung werden für gleiche und gleich wirkende Teile dieselben Bezugsziffern verwendet.

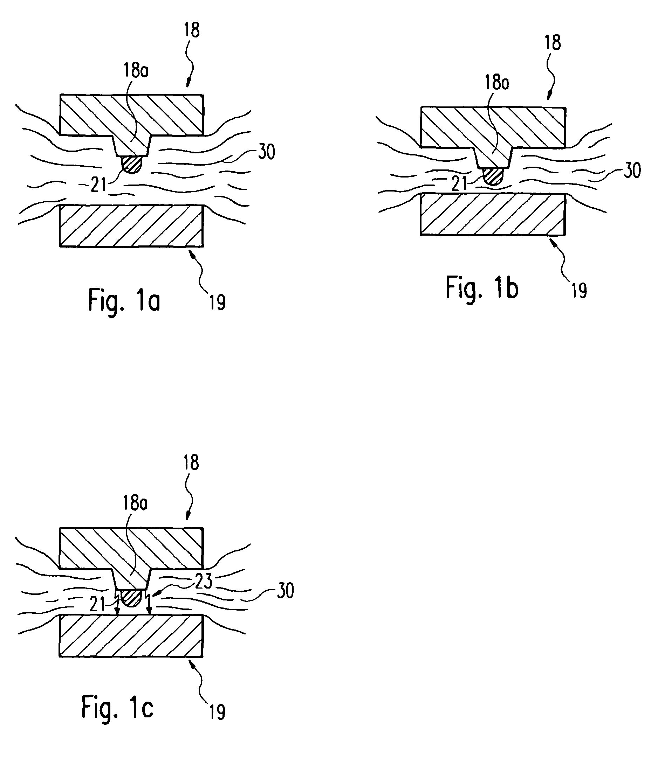

Fig. 1a zeigt eine Vorderansicht im Schnitt einer erfindungsgemäßen Elektrodenanordnung während einer Koagulationsphase in einer ersten Ausführungsform. Dabei sind zwei gegenüberliegende Elektrodenteile 18, 19 dargestellt, wobei ein Elektrodenteil 18 einen Schneidabschnitt 18a und einen isolierenden Abschnitt 21 als ein Distanzelement aufweist. Das Distanzelement bildet in dieser Ausführungsform eine Vorrichtung 20 zur Vermeidung eines Kurzschlusses aus. Zwischen den Elektroden ist ein zu behandelndes Gewebe 30 eingeklemmt.

Der isolierende Abschnitt 21 verhindert einen unerwünschten Kurzschluss zwischen den Elektrodenteilen 18, 19 bei einem Zusammenführen der Branchen 11, 12 und kann sowohl linienförmig als auch punktförmig ausgebildet sein. Das linienförmig ausgebildete Distanzelement zieht sich dann beispielsweise in Richtung des Branchenverlaufs, mittig an dem Elektrodenteil angeordnet, über das gesamte Elektrodenteil und bildet demgemäß eine Kante aus. Vorteilhafterweise wird damit die gleichmäßige Ausbildung eines Lichtbogens ermöglicht und ein ebenmäßiger Schnitt gewährleistet. Ein punktförmig ausgebildetes Distanzelement ist einfach herstellbar, vermeidet zuverlässig die Entstehung des Kurzschlusses zwischen den Elektrodenteilen und gewährleistet aufgrund Wärmeleitung eine sichere Koagulation auch an einer Kontaktstelle zwischen Gewebe und Distanzelement. Mehrere, auf dem entsprechenden Elektrodenteil, z. B. an den jeweiligen Enden des Elektrodenteils angeordnete, punktförmig ausgebildete Distanzelemente, verhindern zuverlässig den Kurzschluss und beeinträchtigen weder einen Koagulations- noch einen Schneidvorgang. Der Schneidabschnitt 18a ist in jedem Fall linienförmig ausgebildet.

Neben der Vermeidung des Kurzschlusses definiert der Abschnitt 21 eine nach der Koagulationsphase verbleibende Dicke des Gewebes 30, weil er eine vorzeitige Lichtbogenentstehung bei eingestellter Koagulationsspannung aufgrund eines zu geringen Abstandes zwischen den Elektrodenteilen 18, 19 verhindert.

Fig. 1b zeigt die Elektrodenanordnung gemäß Fig. 1a, wobei hier allerdings das Ende der Koagulation dargestellt ist. Gemäß der Fig. 1a und 1b fließt ein Koagulationsstrom über eine gesamte Fläche der Elektrodenteile 18, 18a, 19, so dass das dazwischen befindliche Gewebe 30 koaguliert wird. Aufgrund Wärmeleitung wird auch das Gewebe 30 unter dem isolierenden Abschnitt 21 koaguliert.

Fig. 1c zeigt die oben beschriebene Elektrodenanordnung während einer Schneidphase. Nach Beendigung der Koagulationsphase wird eine für die elektrochirurgische Behandlung benötigte HF-Spannung geringfügig erhöht, so dass zwischen dem Schneidabschnitt 18a und dem gegenüberliegenden Elektrodenteil 19 der Lichtbogen 23 entsteht, der das bereits koagulierte Gewebe 30 nun durchtrennt.

Der Schneidabschnitt 18a ist vorzugsweise als ein sich im Verhältnis zu dem Elektrodenteil 18 verjüngender Bereich an dem Elektrodenteil 18 angeordnet und steht aus diesem hervor. Das Elektrodenteil 18 umfasst dann neben dem Schneidabschnitt 18a einen expliziten Koagulationsabschnitt. Das den Koagulationsabschnitt und den Schneidabschnitt 18a ausbildende Elektrodenteil 18 kann während eines Koagulationsvorganges über seinen gesamten Flächenbereich, d. h. sowohl über den Flächenbereich des Koagulationsabschnittes als auch über den Flächenbereich des Schneidabschnittes 18a, als Koagulationselektrode wirken, während der verjüngt ausgebildete Schneidabschnitt 18a allein für einen späteren Schneidvorgang zur Verfügung steht.

Die Höhe des isolierenden Abschnittes 21 und damit der Abstand von Schneidabschnitt 18a und gegenüberliegendem Elektrodeteil 19 und die zum Schneiden benötigte HF-Spannung sind aufeinander abgestimmt. Die Entstehung des Lichtbogens 23 außerhalb des Schneidabschnittes 18a, also an den restlichen Bereichen des Elektrodenteils 18 wird somit vermieden.

Aufgrund der soeben beschriebenen Elektrodenanordnung ist ein und dasselbe Instrument sowohl zum Koagulieren als auch zum Schneiden verwendbar und ein Instrumentenwechsel kann zugunsten eines zügigen Operationsverlaufs vermieden werden.

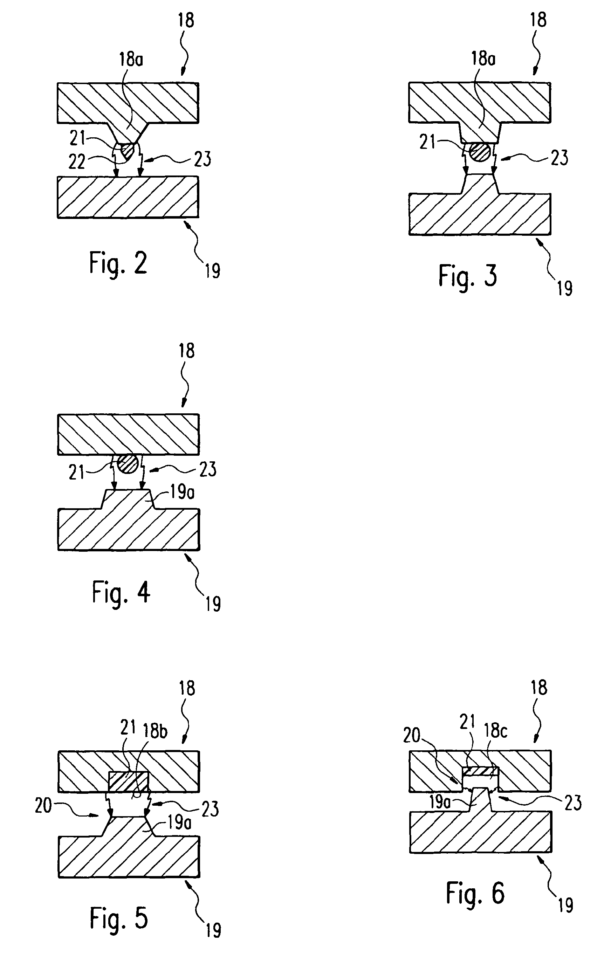

In Fig. 2 ist eine Vorderansicht im Schnitt einer Elektrodenanordnung in einer zweiten Ausführungsform dargestellt. Ein während einer Behandlung zwischen den Elektrodenteilen eingeklemmtes Gewebe ist in dieser Ausführungsform zu Gunsten eine besseren Übersichtlichkeit nicht dargestellt. Gleiches gilt im Übrigen für die Fig. 3 bis 6.

Die Anordnung unterscheidet sich von der in den Fig. 1a bis 1c dargestellten dadurch, dass ein Schneidabschnitt 18a als Kante mit einem dreieckförmigen Querschnitt ausgebildet ist. Aufgrund des sukzessiven Übergangs von einem großen Flächenbereich des Elektrodenteils 18 bis zu deren kantenförmiger Verjüngung ist diese Ausgestaltung in besonderem Maße geeignet, das gesamte Elektrodenteil 18 bei ausreichender Gewebedicke als Koagulationselektrode einzusetzen, weil der gesamte Flächenbereich und das Gewebe in Kontakt bringbar sind. Bei einer geeigneten HF-Spannung entsteht ein Lichtbogen 23 zwischen dem Schneidabschnitt 18a und einem gegenüberliegenden Elektrodenteil 19.

Der isolierende Abschnitt 21 weist in diesem Falle eine spitz zulaufende Form auf, um ggf. auch ein mechanisches Schneiden zu ermöglichen, d. h., das Distanzelement weist eine explizite Schneidkante 22 auf. Mit entsprechender Kraftaufbringung durch den Chirurgen kann dann, nach einem Koagulationsvorgang und ohne einen Instrumentenwechsel vornehmen zu müssen, das Gewebe vollständig durchtrennt werden. Ohne Nutzung eines Lichtbogens wird eine besonders sanfte Behandlung des Gewebes ermöglicht.

Fig. 3 zeigt eine Vorderansicht im Schnitt einer Elektrodenanordnung in einer dritten Ausführungsform. Dabei ist sowohl an einem Elektrodenteil 18 ein Schneidabschnitt 18a als auch an einem Elektrodenteil 19 ein Schneidabschnitt 19a ausgebildet. Ein isolierender Abschnitt 21 ist unmittelbar unterhalb des Schneidabschnittes 18a und symmetrisch zu den Schneidabschnitten 18a, 19a angeordnet. Eine symmetrische Anordnung der zusammenwirkenden Abschnitte 18a, 21, 19a gewährleistet einen gleichmäßig verlaufenden Lichtbogen 23 an Randbereichen des isolierenden Abschnitts 21, so dass ein ebenmäßiger Schnittverlauf ermöglicht wird. Der als ein Distanzelement fungierende isolierende Abschnitt 21 ist kleiner als die Schneidabschnitte 18a, 19a ausgelegt, um die Entstehung des Lichtbogens 23 nicht zu behindern. Aufgrund der an den Elektrodenteilen 18, 19 angebrachten schmalen Schneidabschnitte 18a, 19a ist der Schnittverlauf äußerst präzise definierbar.

Eine äußerst einfache Ausführungsform einer Elektrodenanordnung ist in Fig. 4 gezeigt. Dabei weist nur ein Elektrodenteil 19 einen Schneidabschnitt 19a auf, während an einem dem Elektrodenteil 19 gegenüberliegenden Elektrodenteil 18 lediglich ein isolierender Abschnitt 21 ausgebildet ist. Aufgrund des in Richtung des Schneidabschnittes 19a entstehenden Lichtbogens 23 ist in dieser Ausführungsform auf besonders einfache Weise eine exakte Schnittlinie definierbar.

Fig. 5 zeigt eine Vorderansicht im Schnitt einer Elektrodenanordnung, bei der ein Elektrodenteil 18 einen innerhalb des Elektrodenteils 18 ausgebildeten isolierenden Abschnitt 21 aufweist, wobei der isolierende Abschnitt 21 bündig mit einer Elektrodenfläche 18b abschließt. An einem gegenüberliegenden Elektrodenteil 19 ist ein zweiter Schneidabschnitt 19a als ein Distanzelement vorgesehen. Der isolierende Abschnitt 21 und der Schneidabschnitt 19a wirken in dieser Ausführungsform als Vorrichtung 20 zur Vermeidung eines Kurzschlusses. Vorteilhafterweise wird auch hier eine präzise Schnittführung vorgegeben, da ein Lichtbogen 23 zwischen dem eingearbeiteten Abschnitt 21 und dem Schneidabschnitt 19a entsteht. Der isolierende Abschnitt 21 ist bei dieser Ausführungsform vor Stößen oder dergleichen mechanischen Belastungen und im Wesentlichen auch vor dem Lichtbogen 23 geschützt.

Fig. 6 zeigt eine ähnliche Ausgestaltung einer Elektrodenanordnung, wie sie in Fig. 5 dargestellt ist. Ein isolierender Abschnitt 21 ist hier allerdings vertieft in einem entsprechenden Elektrodenteil 18 ausgebildet, so dass an dem Elektrodenteil 18 eine Aussparung 18c ausgebildet ist. Ein an einem gegenüberliegenden Elektrodenteil 19 ausgebildeter Schneidabschnitt 19a ist mindestens teilweise in der Aussparung 18c versenkbar, so dass sich während eines Schneidvorganges ein Lichtbogen 23 innerhalb der Aussparung 18c hin zu dem Schneidabschnitt 19a ausbilden kann. Umliegendes Gewebe kann so vor Verbrennungen geschützt werden, gleichzeitig ist eine präzise Schnittlinie definierbar.

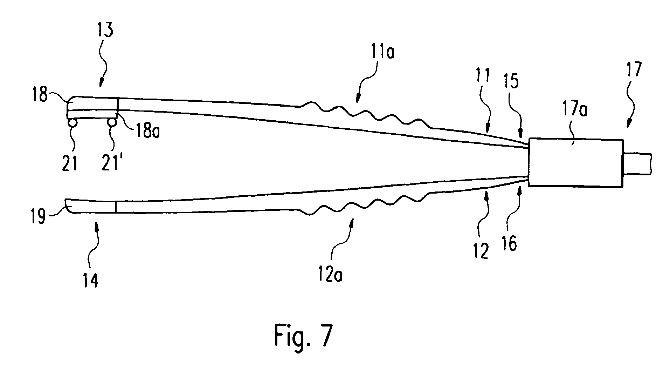

Fig. 7 zeigt ein vollständig abgebildetes elektrochirurgisches Instrument 10 mit einer erfindungsgemäßen Elektrodenanordnung. In der Abbildung sind mit den Bezugsziffern 11 und 12 zwei Branchen des elektrochirurgischen Instruments 10 bezeichnet. Diese Branchen 11, 12 weisen mit Elektrodenteilen 18, 19 versehen distale Enden 13, 14 auf, wobei sich die Elektrodenteile 18, 19 einander gegenüberliegen. Mit Hilfe der Elektrodenteile 18, 19 lässt sich beispielsweise ein Gefäß fassen und durch Zuleitung von hochfrequentem Strom koagulieren bzw. schneiden. Ferner sind Griffteile 11a, 12a vorgesehen, die an jeweilige proximale Enden 15, 16 der Branchen 11, 12 anschließen. Die proximalen Enden 15, 16 der Klemmenteile 11, 12 enden in einem Anschlusselement 17a von Stromzuführungseinrichtungen 17. Die Stromzuführungseinrichtungen 17 dienen zum Anschließen des elektrochirurgischen Instruments 10 an einen (nicht dargestellten) HF-Generator, der eine HF-Spannung erzeugt, so dass ein HF-Strom beispielsweise durch in dem Instrument 10 laufende elektrische Leitungen (nicht gezeigt) den Elektrodenteilen 18, 19 zugeführt werden kann.

An dem Elektrodenteil 18 ist ein kantenförmiger Schneidabschnitt 18a ausgebildet. Dieser trägt zwei als punktförmige Distanzelemente ausgebildete isolierende Abschnitte 21, 21'. Die an den jeweiligen Enden des Elektrodenteils 18 bzw. an dem Schneidabschnitt 18a angeordneten Distanzelemente verhindern zuverlässig den Kurzschluss und beeinträchtigen weder einen Koagulations- noch einen Schneidvorgang.

Um eine hohe Beständigkeit des isolierenden Abschnittes gegenüber einem Lichtbogen zu erzielen, ist der Abschnitt vorzugsweise aus einem abbrandfestem Material ausgebildet. Eine hohe Verschleißfestigkeit ist insbesondere durch die Verwendung keramischer Materialien gegeben.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: Elektrochirurgisches Instrument
- 11: Klemmenteil, Branche
- 11a: Griffteil
- 12: Klemmenteil, Branche
- 12a: Griffteil
- 13: Distales Ende
- 14: Distales Ende
- 15: Proximales Ende
- 16: Proximales Ende
- 17: Stromzuführungseinrichtungen
- 17a: Anschlusselement
- 18: Elektrodenteil
- 18a: Schneidabschnitt
- 18b: Elektrodenfläche
- 18c: Aussparung
- 19: Elektrodenteil
- 19a: Schneidabschnitt
- 20: Vorrichtung zur Vermeidung eines Kurzschlusses
- 21, 21': Isolierender Abschnitt
- 22: Schneidkante
- 23: Lichtbogen
- 30: Gewebe

## Patentansprüche

1. Elektrochirurgisches Instrument mit
- zwei gelenkig miteinander verbundenen Branchen (11, 12), die entsprechend einem Klemmwerkzeug betätigbar sind,
- Elektrodenteilen (18, 19) an distalen Enden (13, 14) der Branchen (11, 12) zum Fassen von Gewebe (30) und zum Durchleiten eines Koagulationsstromes und eines Schneidstromes durch das Gewebe (30) zum Koagulieren und Schneiden des Gewebes,
- Stromzuführungseinrichtungen (17) zum Zuführen des Koagulationsstromes und des Schneidstromes zu den Elektrodenteilen (18, 19) von einem HF-Generator und
- mindestens einer an den Elektrodenteilen (18, 19) ausgebildeten Vorrichtung (20) zur Vermeidung eines Kurzschlusses, die derart angebracht und ausgebildet ist, dass die Elektrodenteile (18, 19) sich nicht berühren können, wobei die Vorrichtung (20) zur Vermeidung eines Kurzschlusses mindestens einen isolierenden Abschnitt (21) aufweist,
**dadurch gekennzeichnet, dass**
- ein Elektrodenteil (18, 19) einen Koagulationsabschnitt und einen aus dem Koagulationsabschnitt hervorstehenden Schneidabschnitt (18a, 19a) umfasst, die jeweils einen Flächenbereich ausbilden, wobei die Flächenbereiche des Koagulationsabschnittes und des Schneidabschnittes (18a, 19a) als Koagulationselektrode wirken und für den Schneidvorgang mittels eines Lichtbogens (23) ausschließlich der Schneidabschnitt (18a) zur Verfügung steht, dass
- der isolierende Abschnitt (21) auf dem Schneidabschnitt (18a, 19a) oder auf dem dem Schneidabschnitt (18a, 19a) gegenüberliegenden Elektrodenteil (19, 18) angeordnet ist, und dass
- der isolierende Abschnitt (21) als Distanzelement ausgebildet ist, wobei seine Höhe den Abstand zwischen dem Schneidabschnitt (18a) und dem gegenüberliegenden Elektrodenteil (18,19) bei zusammengeführten Branchen bestimmt.

2. Elektrochirurgisches Instrument nach einem der vorhergehenden Ansprüche,
**dadurch gekennzeichnet, dass**
an den isolierenden Abschnitt (21) angrenzend mindestens ein erster Schneidabschnitt (18a) an mindestens einem der Elektrodenteile (18) derart angeordnet ist, dass bei einer Erhöhung der Spannung des Koagulationsstromes von dem ersten Schneidabschnitt (18a) ausgehend der Lichtbogen (23) zum Durchtrennen des Gewebes (30) entsteht.

3. Elektrochirurgisches Instrument nach einem der vorhergehenden Ansprüche,
**dadurch gekennzeichnet, dass**
an dem Elektrodenteil (19), das dem Elektrodenteil (18) mit dem isolierenden Abschnitt (21) gegenüberliegt, ein zweiter Schneidabschnitt (19a) ausgebildet ist.

4. Elektrochirurgisches Instrument nach Anspruch 3,
**dadurch gekennzeichnet, dass**
der isolierende Abschnitt (21) symmetrisch zum ersten Schneidabschnitt (18a) und/oder zu dem zweiten Schneidabschnitt (19a) an den jeweiligen Elektrodenteilen (18, 19) angeordnet ist.

5. Elektrochirurgisches Instrument nach einem der vorhergehenden Ansprüche,
**dadurch gekennzeichnet, dass**
das Distanzelement derart ausgebildet ist, dass ein mechanisches Schneiden durchführbar ist.

6. Elektrochirurgisches Instrument nach einem der vorhergehenden Ansprüche,
**dadurch gekennzeichnet, dass**
der isolierende Abschnitt (21) aus einem abbrandfesten Material ausgebildet ist.

7. Elektrochirurgisches Instrument nach einem der vorhergehenden Ansprüche,d a **durch gekennzeichnet**, dass
der isolierende Abschnitt (21) aus einer Keramik ausgebildet ist.

## Claims

1. Electrosurgical instrument with
- two branches (11, 12), which are connected to each other in an articulated manner and can be actuated in the manner of a gripping tool,
- electrode parts (18, 19) at distal ends (13, 14) of the branches (11, 12), for grasping tissue (30) and for conducting a coagulating current and a cutting current through the tissue (30) in order to coagulate and cut the tissue,
- current-supplying devices (17) for supplying the coagulating current and the cutting current to the electrode parts (18, 19) from an HF generator, and
- at least one device (20) for avoiding a short circuit, which device (20) is formed on the electrode parts (18, 19) and is arranged and designed in such a way that the electrode parts (18, 19) cannot touch each other, wherein the device (20) for avoiding a short circuit has at least one insulating portion (21),
**characterized in that**
- an electrode part (18, 19) comprises a coagulating portion and, protruding from the coagulating portion, a cutting portion (18a, 19a), which portions each form a surface area, wherein the surface areas of the coagulating portion and of the cutting portion (18a, 19a) act as coagulating electrode, and the cutting portion (18a) is available exclusively for the cutting procedure by means of an electric arc (23), **in that**
- the insulating portion (21) is arranged on the cutting portion (18a, 19a) or on the electrode part (19, 18) lying opposite the cutting portion (18a, 19a), and **in that**
- the insulating portion (21) is designed as a spacer element, its height determining the distance between the cutting portion (18a) and the opposite electrode part (18, 19) when the branches are brought together.

2. Electrosurgical instrument according to one of the preceding claims, **characterized in that**, adjoining the insulating portion (21), at least a first cutting portion (18a) is arranged on at least one of the electrode parts (18) in such a way that, when the voltage of the coagulating current increases, the electric arc (23) for cutting through the tissue (30) issues from the first cutting portion (18a).

3. Electrosurgical instrument according to one of the preceding claims, **characterized in that** a second cutting portion (19a) is formed on the electrode part (19) lying opposite the electrode part (18) with the insulating portion (21).

4. Electrosurgical instrument according to Claim 3, **characterized in that** the insutating portion (21) is arranged symmetrically with respect to the first cutting portion (18a) and/or to the second cutting portion (19a) on the respective electrode parts (18, 19).

5. Electrosurgical instrument according to one of the preceding claims, **characterized in that** the spacer element is designed such that mechanical cutting can be carried out.

6. Electrosurgical instrument according to one of the preceding claims, **characterized in that** the insulating portion (21) is made of a material resistant to arc erosion.

7. Electrosurgical instrument according to one of the preceding claims, **characterized in that** the insulating portion (21) is made of a ceramic material.

## Revendications

1. Instrument électrochirurgical doté :
de deux branches (11, 12) reliées entre elles de façon articulée et pouvant être actionnées conformément à un outil de serrage ;
de parties d'électrode (18, 19) positionnées aux extrémités distales (13, 14) des branches (11, 12) de façon à saisir le tissu (30) et à guider un courant de coagulation et un courant de coupe à travers le tissu (30) afin de faire coaguler et de couper le tissu ;
de dispositifs de conduction de courant (17) servant à conduire le courant de coagulation et le courant de coupe jusqu'aux parties d'électrode (18, 19) en partant d'un générateur HF ; et
d'au moins un dispositif (20) réalisé au niveau des parties d'électrode (18, 19) servant à éviter un court-circuit, ledit dispositif étant disposé et réalisé de telle sorte que les parties d'électrode (18, 19) ne peuvent pas se toucher, le dispositif (20) servant à éviter un court-circuit comportant au moins une section isolante (21) ;
**caractérisé en ce que** :
- une partie d'électrode (18, 19) comprend une section de coagulation et une section de coupe (18a, 19a) saillante à partir de la section de coagulation formant respectivement une région superficielle, les régions superficielles de la section de coagulation et de la section de coupe (18a, 19a) agissant comme une électrode de coagulation et uniquement la section de coupe (18a) étant mise à disposition pour réaliser le processus de coupe par le biais d'un arc lumineux (23) ;
- la section isolante (21) est disposée sur la section de coupe (18a, 19a) ou sur la partie d'électrode (19, 18) opposée à la section de coupe (18a, 19a) ; et
- la section isolante (21) prend la forme d'un élément d'écartement, sa hauteur définissant la distance entre la section de coupe (18a) et la partie d'électrode (18, 19) opposée lorsque les branches sont guidées conjointement.

2. Instrument électrochirurgical selon selon l'une quelconque des revendications précédentes, **caractérisé en ce qu'**au moins une première section de coupe (18a) connexe à la section isolante (21) est disposée au niveau d'au moins une des parties d'électrode (18) de telle sorte qu'en cas d'augmentation de la tension du courant de coagulation en partant de la première section de coupe (18a), l'arc lumineux (23) apparaît pour couper le tissu (30) en deux.

3. Instrument électrochirurgical selon l'une quelconque des revendications précédentes, **caractérisé en ce qu'**une deuxième section de coupe (19a) est réalisée au niveau de la partie d'électrode (19) qui est opposée à la partie d'électrode (18) avec la section isolante (21).

4. Instrument électrochirurgical selon la revendication 3, **caractérisé en ce que** la section isolante (21) est disposée de façon symétrique par rapport à la première section de coupe (18a) et/ou à la deuxième section de coupe (19a) au niveau des parties d'électrode (18, 19) respectives.

5. Instrument électrochirurgical selon l'une quelconque des revendications précédentes, **caractérisé en ce que** l'élément d'écartement est réalisé de telle sorte qu'une coupe mécanique peut être réalisée.

6. Instrument électrochirurgical selon l'une quelconque des revendications précédentes, **caractérisé en ce que** la section isolante (21) est réalisée à partir d'un matériau ignifuge.

7. Instrument électrochirurgical selon l'une quelconque des revendications précédentes, **caractérisé en ce que** la section isolante (21) est réalisée en céramique.
